# EUROPÄISCHE PATENTSCHRIFT

(11) **EP 4 582 529 B1**
(45) Veröffentlichungstag und Bekanntmachung des Hinweises auf die Patenterteilung: **18.03.2026**
(21) Anmeldenummer: 24150727.6
(22) Anmeldetag: 08.01.2024
(51) Int. Cl.: G01N 35/04, C12M 1/00, B01L 1/02, C12M 3/00, C12M 3/06, G01N 35/00, C12M 1/32

(54) **INKUBATOR**
INCUBATOR
INCUBATEUR

(43) Veröffentlichungstag der Anmeldung: 09.07.2025
(73) Patentinhaber: BdelloRob GmbH, 70180 Stuttgart (DE)
(72) Erfinder: Feigel, Burkhard Johannes, 70180 Stuttgart (DE)
(74) Vertreter: Christ, Niko

(56) Entgegenhaltungen:
- EP-A1- 3 196 287
- EP-A1- 4 036 212
- EP-A1- 4 253 523
- EP-B1- 2 232 175
- WO-A1-2014/161656
- WO-A1-2022/263651
- DE-A1- 102006 003 117
- DE-A1- 102020 102 758
- DE-B3- 102014 011 941
- US-A1- 2018 320 122
- US-A1- 2018 346 868
- US-A1- 2023 399 600

## Beschreibung

Die vorliegende Erfindung betrifft einen Inkubator, umfassend einen gegenüber einer Umgebung druck- und gasdicht verschließbaren, sowie hinsichtlich der Zusammensetzung der Gase und Feuchtigkeit seiner Atmosphäre anpassbaren Inkubationsraum zur Aufnahme wenigstens einer Mikrotiterplatte, in dem eine Hub-Greifeinheit zum Transport der wenigstens einen Mikrotiterplatte innerhalb des Inkubationsraums beweglich gelagert ist, wobei der Inkubationsraum über eine Schleusenkammer zugänglich ist, welche gegenüber der Umgebung mithilfe einer Außentür und gegenüber dem Inkubationsraum mithilfe einer Innentür verschließbar ist, wobei die Hub-Greifeinheit zwischen einer ersten Position oberhalb der Schleusenkammer und wenigstens einer zweiten Position oberhalb einer Lagerstelle für die wenigstens eine Mikrotiterplatte verfahrbar ist. Ein solcher Inkubator ist bereits aus der DE 10 2020 102758 A1, vorbekannt. Hierin wird ein Isolator/Inkubator offenbart, sowie ein Verfahren zum automatisierten Keimmonitoring im Inkubator, welcher hier eine Transferschleuse aufweist. Dabei wurde in dieser Schrift ein Roboter im Inkubator angeordnet, der über einen Greifer zum Aufnehmen der Nährbodenträger verfügt. Ein Nährbodenträger kann dabei vom Roboter in einen freien Nährbodenträgerhalter transferiert werden. Für die Aufnahme wenigstens einer Mikrotitierplatte ist der Inkubationsraum gut geeignet.

Ein derartiger Inkubator ist bereits aus der DE 10 2006 003 117 A1 vorbekannt. Dieses Dokument bezieht sich auf eine Vorrichtung zur Energieübertragung zwischen einem Antriebsbereich und einem räumlich davon getrennten Arbeitsbereich in einem Inkubator. Die Energie von mindestens einer Antriebsvorrichtung wird dabei mittels mindestens eines Übertragungselements und eines zeitlich und/oder räumlich veränderlichen Kraftfelds auf mindestens ein Übertragungselement im Arbeitsbereich übertragen und dort in eine Bewegung umgewandelt.

Allgemein stößt man bei der Konstruktion eines Inkubators auf zahlreiche Probleme. Zunächst sollen für die Zucht und Kultivierung möglichst hilfreiche Umgebungsbedingungen bereitgestellt werden, was sowohl eine erhöhte Temperatur als auch eine erhöhte Feuchtigkeit, sowie eine Gaszusammenstellung bedeuten kann, welche von der Umgebungsluft abweicht. Es ist daher wichtig, einen abgeschlossenen Bereich zur Verfügung zu stellen, in dem diese Bedingungen erfüllt sind. Dies ist der erwähnte Inkubationsraum.

Um zu vermeiden, dass bei stehenden Zellkulturen ein zu geringer Gasaustausch entsteht, ist es bekannt, Schüttler zu verwenden, mit denen die Mikrotiterplatten mit den darin aufgenommenen Zellkulturen bewegt bleiben. Hierdurch wird der Gasaustausch angeregt. Es erhöht sich jedoch die Gefahr einer Austrocknung durch Verdunstung. Durch offene Wasserflächen, sowie gegebenenfalls Ultraschallzerstäuber, kann die Feuchtigkeit erhöht werden. Alle offenen Wasserflächen und Tropfen, welche durch Kondensation entstehen, begünstigen das Wachstum von Pilzen. Letztere sind die größten Feinde einer Kultur mit eukaryotischen Zellen.

Eine übliche Sterilisierung bei 1,2 bar und 180° C für 20 Minuten ist im Inkubator nicht vorgesehen, da dieser nicht ausreichend druckfest ist. Eine Sterilisation mit Wasserstoffperoxid ist als Alternative ebenfalls ausgeschlossen, da es zu Wasser reagiert und dann eine Grundlage für das Wachstum von Pilzen bilden würde. Möglich ist schließlich eine Ozonsterilisation, welche wirksam ist, allerdings aufgrund der frei werdenden Sauerstoffradikale sehr korrosiv wirkt. Erforderlich ist daher bei dieser Vorgehensweise die möglichst weitgehende Verwendung von nicht korrosiven Materialien wie Edelstahl. Dies macht aber die Anordnung von automatisierten Vorrichtungen in dem Inkubator sehr teuer.

Die genannte DE 10 2006 003 117 A1 schlägt daher bereits vor, eine Pipettiervorrichtung vorzusehen, welche durch eine nichtmagnetische Platte hindurch mit einem außenliegenden Roboter bedient werden kann. Nur die notwendigsten Teile liegen damit in dem Inkubationsraum und sind den Sterilisierungen mit Ozon ausgesetzt, während die komplexen Steuerungssysteme sich außerhalb des Inkubationsraums befinden können.

Weiter ist in diesem Sinne aus der WO 2014/161656 A1 eine Manipulatorvorrichtung bekannt, die mit einer Positioniereinrichtung verbunden ist. Diese Vorrichtung besteht aus einem Manipulatorkopf, der eine erste Komponente in Form einer zur mechanischen Verbindung mit der Positioniereinrichtung dienenden Halteeinheit und eine zweite Komponente in Form eines von der Halteeinheit gehaltenen Endeffektors aufweist. Beide Komponenten können permanentmagnetische Eigenschaften haben, die Kopplungseinheit außerhalb des Inkubationsraumes kann gesteuerte Magneten tragen. Der Endeffektor wird durch die Interaktion der beiden Kopplungseinheiten unter Belassung eines definierten Luftspaltes berührungslos von der Halteeinheit gehalten.

Schüttelinkubatoren für Miktrotiterplatten sind derzeit nur manuell beschickbar und nicht für Automatisation geeignet. Eine solche automatisierte Entnahme stellt jedoch einen deutlichen Vorteil dar, da auf diese Weise einzelne Mikrotiterplatten unabhängig von anderen entnommen und darin enthaltene Zellkulturen einer Analyse unterzogen werden können.

Insoweit ist die EP 2 232 175 B1 aus dem Stand der Technik vorbekannt. Dieses Dokument bezieht sich auf ein automatisiertes Lager- und Abrufsystem für die Speicherung von biologischen oder chemischen Proben bei extrem niedrigen Temperaturen. Das System besteht aus einer Tiefkühleinheit mit einem isolierten Gefrierfach, einem Lagergestell innerhalb des Gefrierfachs und einem Roboter, der für den Transport der Probenbehälter innerhalb des Gefrierfachs zuständig ist. Die Robotermotoren sind außerhalb des Gefrierfachs an einer isolierten Tür montiert. Die Leistung wird durch magnetische Kopplungen vom Roboterantriebsmotor auf den Roboter übertragen. Das System kann auch eine Abteilung für eine Röhrenauswahlmaschine auf der Tür enthalten. Zusätzlich kann die Umgebung innerhalb der Röhrenauswahlkammer durch Zuführung von trockenem Gas gesteuert werden, um die Luftfeuchtigkeit zu senken.

Schließlich ist auch der Gegenstand der WO 2022/263651 A1 vorbekannt. Dieses Dokument bezieht sich auf eine Vorrichtung zur Bewegung oder Positionierung eines Objekts, speziell in Branchen wie der Lebensmittel- oder Pharmaindustrie. Das Objekt wird berührungslos auf einer Antriebsfläche bewegt, die durch wenigstens einen Mover realisiert wird, der magnetisch an eine Statoranordnung gekoppelt ist. Die Antriebsfläche ist als eine dichte Grenze bzw. Begrenzungswand eines geschützten Innenraums ausgebildet. Die Stator-Anordnung kann außerhalb des geschützten Innenraums angeordnet sein. Die Vorrichtung kann auch mehrere Kammern aufweisen, die durch eine Zwischenwand getrennt sind. Dies erlaubt die Manipulation von Objekten innerhalb der Kammern und/oder durch eine kleine Zugangsöffnung in der Zwischenwand. Die Anordnung ist konzipiert, um Flexibilität, Effizienz und Sauberkeit in Produktionsumgebungen zu optimieren.

Die Schrift EP 4 036 212 A1 offenbart einen Inkubator für lebende Zellkulturen mit einem Bilderfassungssystem sowie ein Verfahren zur Arbeit mit einem Inkubator. Hierbei kann man die Atmosphäre des Inkubators regeln und mit mindestens einer Beleuchtung, Kameraeinrichtung, mindestens einem Bilderfassungssystem und einer Datenverarbeitungseinrichtung mit Datenspeicher ausstatten. Die Kammer dieses Inkubators ist durch ein mit der Inkubatorkammer beweglich verbundenes Verschlusselement verschließbar, insbesondere mittels eines Scharniers an der Inkubatortür und einer oder mehrerer thermisch isolierter Kammertüren. Weiterhin kann hier der Inkubator eine oder mehrere thermisch isolierte Innentüren aufweisen. Zur Lagerung im Inkubator dient eine Lagerplatte, welche als Regalblecheinsatz und/oder als bewegliche Plattform realisiert ist.

Die Umgebungsbedingungen für eine Entnahme von Proben aus einer kalten und trockenen Umgebung müssen jedoch als nicht vergleichbar mit den Erfordernissen in einem Inkubator angesehen werden. Die Umgebung der WO 2022/263651 A1 ist ebenfalls zwar als Dekontaminationsbereich vorgesehen, geht aber wiederum nicht auf den besonderen Fall des vorliegenden Inkubators ein.

Vor diesem Hintergrund liegt der vorliegenden Erfindung die Aufgabe zu Grunde, einen Inkubator anzugeben, der sowohl für eine effektive Sterilisierung geeignet, als auch vollständig zur Entnahme von Mikrotiterplatten automatisierbar ist.

Gelöst wird diese Aufgabe durch einen Inkubator gemäß den Merkmalen des unabhängigen Anspruchs 1. Sinnvolle Ausgestaltungen eines solchen Inkubators können den sich anschließenden abhängigen Ansprüchen entnommen werden.

Vorgesehen ist insoweit ein Inkubator, umfassend einen gegenüber einer Umgebung druck- und gasdicht verschließbaren, sowie hinsichtlich der Zusammensetzung der Gase und Feuchtigkeit seiner Atmosphäre anpassbaren Inkubationsraum zur Aufnahme wenigstens einer Mikrotiterplatte, in dem eine Hub-Greifeinheit zum Transport der wenigstens einen Mikrotiterplatte innerhalb des Inkubationsraums beweglich gelagert ist. Dieser ist erfindungsgemäß dadurch gekennzeichnet, dass der Inkubationsraum über eine Schleusenkammer zugänglich ist, welche gegenüber der Umgebung mithilfe einer Außentür und gegenüber dem Inkubationsraum mithilfe einer Innentür verschließbar ist, wobei die Hub-Greifeinheit zwischen einer ersten Position oberhalb der Schleusenkammer und wenigstens einer zweiten Position oberhalb einer Lagerstelle für die wenigstens eine Mikrotiterplatte verfahrbar ist.

Mit anderen Worten kann der Inkubator mit einer Schleuse beschickt werden, auf welche direkt von der in dem Inkubationsraum vorgesehenen Hub-Greifeinheit aus zugegriffen werden kann. Dadurch, dass die Hub-Greifeinheit zunächst über der Schleusenkammer positioniert werden kann, ist es möglich, eine eingeschleuste Mikrotiterplatte mit der Hub-Greifeinheit zu fassen und anzuheben. In diesem angehobenen Zustand kann die Hub-Greifeinheit zu einer Lagerstelle für die Mikrotiterplatte verfahren werden, wo sie wieder abgelegt werden kann. Die Mikrotiterplatte verbleibt dort während einer Kultivierungszeit, in der die Umgebungsbedingungen innerhalb des Inkubationsraums für ein Zellwachstum möglichst günstig eingestellt werden. Soll eine Mikrotiterplatte zur Analyse ausgeschleust werden, so kann diese wiederum mithilfe des Hub-Greifers von der Lagerstelle aufgenommen und angehoben und in der entsprechenden Aufnahme in der Schleusenkammer abgelegt werden. Hierdurch wird der Vorgang zunächst innerhalb des Inkubators vollständig automatisierbar.

In konkreter Ausgestaltung kann vorgesehen sein, dass die Innentür auf oder in oder über einem Boden des Inkubationsraums angeordnet und abgedichtet ist und entweder horizontal verschiebbar oder um eine horizontale Achse herum in den Inkubationsraum hinein verschwenkbar ist. Über eine Innentür im Boden kann sichergestellt werden, dass die Hub-Greifeinheit die Mikrotiterplatte unproblematisch in der Schleusenkammer positionieren kann, ohne diese über eine Wandung hinwegheben zu müssen. Im Fall einer Positionierung auf oder über dem Boden kann aber die Lagerstelle der Mikrotiterplatte für den gleichen Effekt entsprechend höher gewählt sein. Die Schleusenkammer kann durch die Innentür abgedichtet werden, um den Einfluss einer Ausschleusung auf das Klima in dem Inkubationsraum möglichst gering zu halten. Erst wenn in der Schleusenkammer im Wesentlichen dasselbe Klima hergestellt wurde, wie in dem Inkubationsraum, wird idealerweise die Innentür geöffnet. Eine Schwenktür stellt dabei eine einfache bauliche Lösung dar, eine horizontal verlaufende Schiebetür ist jedoch ebenfalls sinnvoll realisierbar.

Weiter kann vorgesehen sein, dass die Außentür an einer Außenwandung des Inkubationsraums angeordnet und abgedichtet ist und entweder vertikal verschiebbar oder um eine horizontale Achse herum verschwenkbar ist. Die Außentür wird beim Ausschleusen erst geöffnet, wenn die Innentür bereits verschlossen ist und insoweit das Klima innerhalb des Inkubationsraums nicht durch die offene Schleuse beeinträchtigt werden kann.

Mit besonderem Vorteil können die Innentür und die Außentür selbsttätig öffnend und schließend sein, vorzugsweise durch einen elektrischen oder pneumatischen Antrieb betätigt sein. Dies erlaubt es, neben dem Bereitstellungsvorgang der Mikrotiterplatte auch den Vorgang des Ausschleusens, sowie in umgekehrter Richtung auch den Vorgang des Einschleusens, zu automatisieren.

Insbesondere jedoch für den Fall, dass eine manuelle Beschickung ermöglicht sein soll, kann vorgesehen sein, dass der Außentür eine motorisch entkoppelte Sicherheitstür zugeordnet ist, deren Verschlussposition sensorisch erfasst ist, wobei eine Schließung der Außentür nur dann ermöglicht ist, wenn die Sicherheitstür verschlossen ist. Hierdurch wird im Zusammenhang mit dem Schließen der Außentür zunächst sichergestellt, dass keine Körperteile oder Gegenstände im Bereich der Außentür vorhanden sind. Dies ist ausgeschlossen wenn die zusätzliche Sicherheitstür geschlossen ist, was durch einen Sensor, sei es einen Schaltaktor, einen Reed-Kontakt oder dergleichen mehr, sichergestellt werden kann. Nach dem Verschließen der Sicherheitstür kann die Außentür ebenfalls verschlossen werden, beim Öffnen können Außentür und Sicherheitstür gleichzeitig betätigt werden.

Im Fall der Automatisierung der Außentür, also insbesondere wenn auf die Sicherheitstür verzichtet werden kann, kann im Zug einer automatischen Beschickung eine Fördereinrichtung vorgesehen sein, welche der Schleusenkammer die Mikrotiterplatten selbsttätig zuführt. Eine entsprechend automatisierte Förderstrecke kann insbesondere zwischen dem Inkubator und einer Analyseeinheit aufgebaut sein, so dass Mikrotiterplatten selbsttätig aus dem Inkubator entnommen und der Analyse zugeführt werden können. Nach der Analyse kann in umgekehrter Richtung eine Rückführung in den Inkubator durchgeführt werden.

Für eine Bewegung der Mikrotiterplatte aus der Schleusenkammer heraus kann der Schleusenkammer ein Ausschieber zugeordnet sein, welcher eine Lagerstelle für wenigstens eine Mikrotiterplatte ausbildet und aus der Schleusenkammer heraus durch die Außentür, vorzugsweise mithilfe eines elektrischen oder pneumatischen Antriebs, in eine Zugriffsposition verfahrbar ist. Hierbei kann sich der elektrische oder pneumatische Antrieb im Boden der Schleusenkammer, also unter dem Ausschieber, aber auch in Ausschubrichtung hinter dem Ausschieber befinden.

Mit einigem Vorteil kann vorgesehen sein, dass eine Raumdecke des Inkubationsraums als nichtmagnetische Trennplatte ausgestaltet ist, wobei ein Betätigungsroboter mit einer Steuereinheit zur Steuerung einer Bewegung der Hub-Greifeinheit mit einer Führungseinheit oberhalb der Trennplatte angeordnet ist und die Hub-Greifeinheit mithilfe einer durch die Trennplatte hindurch wirkenden magnetischen oder elektromagnetischen Magnethalterung im Inneren des Inkubationsraums auf der Innenfläche der Trennplatte verschieblich gehalten ist und einen höhenverstellbaren Greifer umfasst. Diese Anordnung erlaubt es, eine Hub-Greifeinheit bereitzustellen, welche nur zu einem minimalen Teil innerhalb des Inkubationsraums aufgenommen ist. Während die Trennplatte vollständig durchgehend und nicht durchbrochen ist, kann die Magnethalterung durch die Trennplatte hindurch an der Führungseinheit gehalten werden, so dass eine Bewegung der Führungseinheit aufgrund der magnetischen Kopplung ein Nachziehen der Magnethalterung bewirkt.

Um dem so genannten Stick-Slip-Effekt zu begegnen, aufgrund dessen die Magnethalterung wegen Haftreibung an der Trennplatte hängenbleiben und dadurch den magnetischen Kontakt zur Führungseinheit verlieren kann, kann zudem vorgesehen sein, dass die Führungseinheit und/oder die Magnethalterung, vorzugsweise beide Teile, Luftauslassdüsen auf einer der Trennplatte zugewandten Fläche zur Ausbildung eines Luftkissens zwischen der Fläche und der Trennplatte aufweisen. Das Luftkissen drückt die beiden Teile dann jeweils von der Trennplatte etwas weg, so dass die Reibung reduziert wird oder ganz entfällt, jedoch nicht so stark, dass der Bereich der gegenseitigen Magnetwirkung überwunden wird. Vielmehr wird ein Gleichgewichtszustand zwischen der Magnetwirkung und der Schwerkraft angestrebt, so dass ein möglichst konstanter Abstand zwischen dem jeweiligen Teil und der Trennplatte eingenommen wird. Da die Schwerkraft der Führungseinheit auf die Trennplatte zu und die Schwerkraft der Magnethalterung von der Trennplatte weg wirkt, muss das Luftkissen der Führungseinheit stärker sein als das der Magnethalterung. Dies kann beispielsweise über eine Abstandsregelung durch die Einstellung der Magnetstärke an der oberen Kopplungseinheit eingestellt werden, zumal in Abhängigkeit von dem Gewicht einer angehobenen Mikrotiterplatte das Gewicht der Magnethalterung variieren kann.

Ein weiterer Lösungsansatz ist der Einsatz des SupraMotion-Prinzips der Firma Festo, welches aber sehr teuer und schwer ist. Dies ist jedoch eine eingeplante Option in der High End Version des beschriebenen Systems.

Ebenfalls kann die wirkende Vertikalkraft redziert werden. Die Vertikalkraft wird über die Stärke der Magnete bestimmt. Eine sorgfältige Berechnung der Magnetkraft und die Vermeidung einer Überdimensionierung kann das Problem ebenfalls lösen, was auch etwa durch den Einsatz eines elektronisch geregelten Elektromagneten möglich ist. Dieser wäre auf der außen liegenden Seite des Inkubationsraums anzuordnen, um Korrosionen zu vermeiden.

Besonders vorteilhaft kann es sein, wenn die Luft zur Erzeugung des Luftkissens aufseiten der Magnethalterung aus dem Inkubationsraum angesaugt und vorzugsweise durch ein HEPA-Filter geleitet und/oder durch eine Peltier-Kühlung gekühlt wird. Neben der Erzeugung des gewünschten Luftkissens kann auf diese Art und Weise eine Luftzirkulation mit Luftreinigung erzeugt werden, ohne dass die Gefahr besteht, Keime oder Sporen von außen in den Inkubationsraum zu transportieren. Ein weiterer Vorteil ist, dass zur Luftumwälzung im Inkubator kein Ventilator oder ähnliches benötigt wird. Nicht zuletzt sei erwähnt, dass in den zirkulierenden Luftstrom eine Peltierkühlung eingebaut werden kann, welche zu einer Temperatursenkung im Inkubationsraum beitragen kann, sollte dies erforderlich sein.

In einer ersten Ausgestaltungsform kann die Hub-Greifeinheit einen elektrisch betriebenen Greifer aufweisen, welcher über die Magnethalterung induktiv mit einer Betriebsspannung versorgt ist. Auf diese Weise kann die Energie und können auch die Steuersignale die Trennplatte passieren, ohne dass die Trennplatte hierzu durchbrochen werden muss. Eine separate Anbindung der Hub-Greifeinheit kann entfallen und sie kann sehr kompakt gebaut werden. Allerdings müssen dann sowohl die Steuerelektronik als auch die Aktoren korrosionsfest konstruiert werden, was vergleichsweise aufwändig ist.

Daher sieht eine alternative zweite Ausgestaltungsform vor, dass die Hub-Greifeinheit einen pneumatisch betriebenen Greifer aufweist, welcher mithilfe pneumatischer Schlauchleitungen, welche an wenigstens einer Außenwandung, dem Boden oder der Trennplatte in den Inkubationsraum münden und von außerhalb des Inkubationsraums beschickt sind, mit Druckluft versorgt ist. Hierbei vermischt sich die von außen zugeführte Druckluft nicht mit der Luft innerhalb des Inkubationsraums, sondern wird separat zu- und abgeleitet.

Mit besonderem Vorteil kann vorgesehen sein, dass die Trennplatte als Glasplatte, vorzugsweise aus Borosilikatglas, ausgestaltet ist. Borosilikatglas weist neben dem typischen Hauptbestandteil Siliziumdioxid große Mengen Bortrioxid auf, welches das Glas korrosionsbeständig macht. Für eine Bewegung von Führungseinheit und Magnethalterung stellt das Glas eine glatte und belastbare Oberfläche bereit und erlaubt es zudem, den Beladungszustand des Inkubators von außen zu erfassen.

Mit besonderem Vorteil erlaubt dies jedoch auch, dass der Inkubationsraum durch die Glasplatte hindurch mithilfe einer außerhalb des Inkubationsraums angeordneten Beleuchtungseinrichtung beleuchtet ist. Besonders für die Aufzucht von phototrophen Organismen kann dies nützlich sein, da diese das Licht als Energiequelle nutzen und die Beleuchtungseinrichtung hierdurch nicht innerhalb des Inkubationsraums angeordnet werden muss. Im Fall einer seitlichen Beleuchtung besteht zudem das Problem einer Abschattung in den Kavitäten der Mikrotiterplatten, so dass eine Beleuchtung von oben optimal ist. Als Beleuchtungseinrichtung eignet sich besonders eine LED-Platte mit Hochleistungs-LEDs in warmweiß. Bei der Anordnung der Beleuchtung oberhalb der Glasplatte und außerhalb des Inkubationsraums ist die Abführung der Wärme, welche durch die Beleuchtungseinheit entsteht, leicht abzuführen, ohne dass die Temperatur im Inkubationsraum beeinträchtigt wird.

Um eine ausreichende Haltbarkeit der Hub-Greifeinheit sicherzustellen, kann mit besonderem Vorteil vorgesehen sein, dass diese zumindest im Wesentlichen aus einem nichtkorrosiven Material, vorzugsweise aus Edelstahl, hergestellt ist. Edelstahl ist zwar vergleichsweise kostspielig, aber sowohl langlebig und wartungsarm, als auch korrosionsfest. Die Herstellung komplexer Geräte aus Edelstahl gestaltet sich für den Fachmann unproblematisch.

Hinsichtlich der Handhabung der Mikrotiterplatten kann die Erfindung bevorzugt weiterentwickelt werden. So kann vorgesehen sein, dass der Schleusenkammer Mittel zur selbsttätigen Identifikation einzelner Mikrotiterplatten zugeordnet sind, insbesondere ein Barcodeleser oder ein Nahfeldkommunikationsleser. Eine ankommende Mikrotiterplatte, die beispielsweise nach einer Analyse des Zustands darin gezüchteter Zellkulturen in dem Inkubator aufgenommen wird, kann an der Schleusenkammer hierdurch identifiziert werden, so dass innerhalb eines Steuerungssystems die Information vorgehalten wird, wo sich welche Probe befindet.

Die Integration des essentim Sensor-Systems (www.essentim.com) ist ebenfalls eine Möglichkeit der Identifikation und Bewegungserfassung im und außerhalb des Inkubators.

Ebenfalls kann vorgesehen sein, dass dem Boden des Inkubationsraums wenigstens ein Mikrotiterplattenschüttler, vorzugsweise eine Mehrzahl von rasterförmig angeordneten Mikrotiterplattenschüttlern, zugeordnet ist, wobei der wenigstens eine Mikrotiterplattenschüttler vorzugsweise im Rundlauf und/oder im Längslauf betreibbar ist. Bei ruhenden Proben kann sich im Lauf der Zeit eine Kahmhaut bilden. Es ist daher bekannt, die Proben mit einem Mikrotiterplattenschüttler bewegt zu halten. Hierbei handelt es sich üblicherweise um eine Platte, auf der Behältnisse aufgestellt sind und die sich bewegt. Die Erfindung kann vorsehen, dass der wenigstens eine Mikrotiterplattenschüttler auf einer Basisplatte aufgesteckt ist, die zur Reinigung und/oder Desinfektion mit sämtlichen Mikrotiterplattenschüttlern als Ganzes aus dem Inkubationsraum entnehmbar ist, sowie dass der wenigstens eine Mikrotiterplattenschüttler eine Lagerstelle zur zentrierten und vorzugsweise kraft- oder reibschlüssigen Aufnahme der wenigstens einen Mikrotiterplatte aufweist, wobei vorzugsweise die Lagerhöhe einer Mikrotiterplatte in der Lagerstelle des wenigstens einen Mikrotiterplattenschüttlers der Lagerhöhe der Mikrotiterplatte in der Schleusenkammer zumindest näherungsweise entspricht. Hierdurch kann jede Mikrotiterplatte für sich in einer geeigneten Frequenz und Richtung geschüttelt werden und wird im Sinne eines automatisierten Betriebs, insbesondere einer automatisierten Beschickung in einer definierten Aufnahme zentriert und fixiert.

Zudem kann vorgesehen sein, dass dem Boden des Inkubationsraums wenigstens ein Analysator zur Auswertung von Proben, etwa zur Messung der optischen Dichte, in einer auf einem dem Analysator im Boden freigehaltenen Fenster abgestellten Mikrotiterplatte zugeordnet ist. Soweit für die jeweilige Anwendung eine solche Analyse ausreichend ist, kann darauf verzichtet werden, die Mikrotiterplatte zur Analyse auszuschleusen, so dass gegebenenfalls auch auf die Schleuse ganz verzichtet werden kann, ansonsten können das Fenster und der Analysator vorteilhafterweise neben der Schleuse platziert werden. Es genügt hierfür also, wenn die Hub-Greifeinheit die Mikrotiterplatte zwischen ihrer jeweiligen Lagerstelle und dem Fenster des Analysators hin und herbewegt. Das Fenster als solches kann vorteilhafterweise wie die Trennplatte aus Borosilikatglas hergestellt sein, um diese möglichst haltbar gegenüber der Atmosphäre in dem Inkubationsraum zu machen.

Die vorstehend beschriebene Erfindung wird im Folgenden anhand eines Ausführungsbeispiels näher erläutert.

Es zeigen
- Figur 1: einen erfindungsgemäßen Inkubator in einer schematischen Draufsicht auf einen Querschnitt durch den Inkubationsraum,
- Figur 2: den Inkubator gemäß Figur 1 in einer seitlichen Schnittdarstellung durch die Schleuse,
- Figur 3: die Hub-Greifeinheit des Inkubationsraums in einer perspektivischen Darstellung, sowie
- Figur 4: ein Detail der Magnethalterung der Hub-Greifeinheit gemäß Figur 3 und der zugehörigen Führungseinheit in einer seitlichen Querschnittsdarstellung.

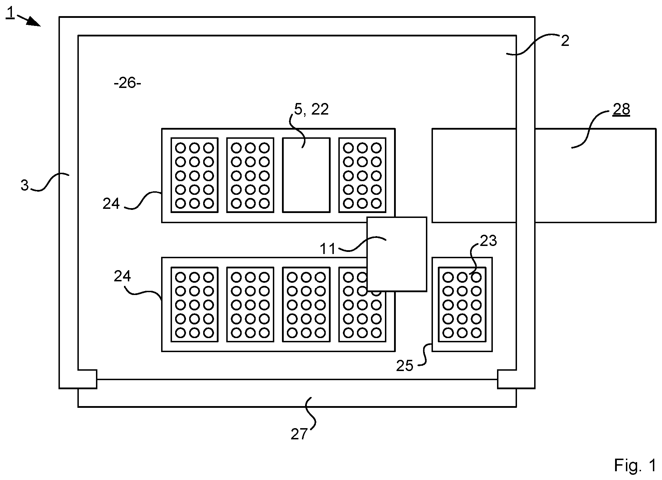

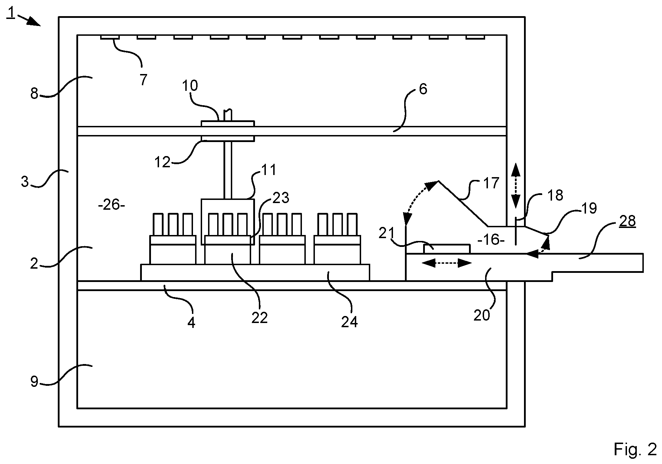

Figur 1 und Figur 2 zeigen einen Inkubator 1 für die Kultivierung von eukaryontischen Zellen und anderen Zelltypen mit geregelter CO2-Begasung und Befeuchtung, welcher einen luftdicht abgedichteten Inkubationsraum 2 umfasst, in dem unter vorgegebenen Bedingungen in den Kavitäten von Mikrotiterplatten 23 aufgenommene Proben von eukaryotischen Zell- und/oder Bakterienkulturen aufgenommen sind. Während Figur 1 eine Draufsicht auf einen Querschnitt durch den Inkubationsraum 2 zeigt, umfasst Figur 2 auch die darüber und darunter angeordnete Roboterkammer 8 und die Steuerkammer 9. In der Roboterkammer 8 ist ein Roboter angeordnet, welcher die Hub-Greifeinheit 11 führt, wie nachfolgend noch beschrieben wird. Auf die genaue Anordnung des Roboters kommt es vorliegend nicht an, so dass die Roboterkammer 8 insoweit leer dargestellt ist. Entsprechendes gilt für die Steuerkammer 9, in welcher eine Steuereinheit angeordnet sein kann, sowie bedarfsweise auch weitere benötigte Einrichtungen wie insbesondere ein Analysator 25. Für eine Zugänglichkeit des Inkubationsraums 2, welche von isolierten Wandungen eingeschlossen ist, sorgt eine Gehäusetür 27, welche eine doppelte Glasscheibe aus Borosilikatglas aufweist.

Die Mikrotiterplatten 23 werden während der Zucht an einer Lagerstelle 5 platziert, welche als Induktionsschüttler 22 ausgestaltet ist. Immer vier Mikrotiterplattenschüttler 22 sind gemeinsam in einer Dockingstation 24 aufgenommen und können jeweils eine Mikrotiterplatte 23 lagern und in Bewegung halten. Hierfür ist unterhalb der Lagerstelle 5 eine hier nicht näher dargestellte Mimik vorgesehen, welche den Mikrotiterplattenschüttler 22 im Rundlauf oder im Längslauf oszillierend bewegt. Die Proben werden hierdurch in Bewegung gehalten, um eine Durchmischung mit Umgebungsgas zu ermöglichen und die Bildung einer Kahmhaut zu vermeiden. Um aufgrund des hierdurch erhöhten Luftkontakts eine Austrocknung zu vermeiden, ist die Luftfeuchtigkeit innerhalb des Inkubationsraums 2 erhöht. Temperatur und Luftfeuchtigkeit werden über die erwähnte Steuereinheit gesteuert, die zudem über den ebenfalls erwähnten Roboter eine Hub-Greifeinheit 11 steuert, welche wiederum die Mikrotiterplatten 23 an einer ersten Lagerstelle 5 auf Anforderung selbsttätig greifen und zu einer Lagerstelle 21 in einer Schleuse 28 verbringen kann.

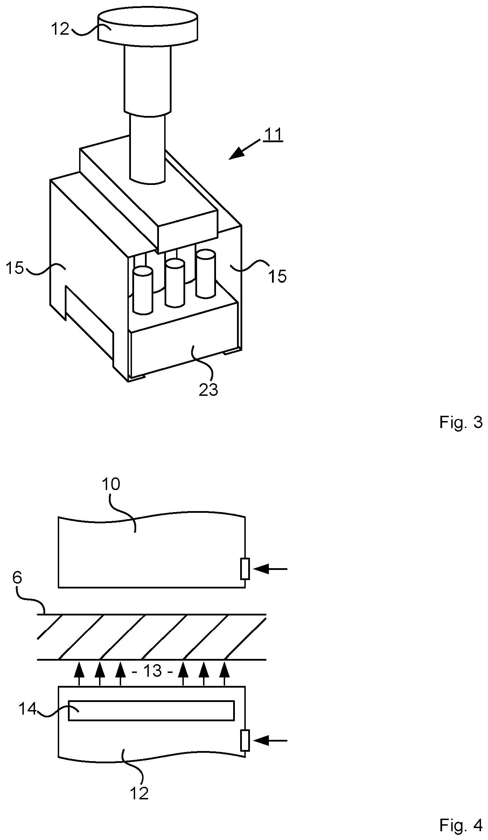

Der Inkubationsraum 2 als Teil des gesamten Inkubators 1 ist von einer Trennplatte 6 übergriffen, welche aus Borosilikatglas hergestellt ist und über der Beleuchtungseinrichtungen 7 angeordnet sind, mit denen phototrophe Zell- und/oder Bakterienkulturen mit Licht, und dadurch mit Energie, versorgt werden können. Auf der Außenseite des Inkubationsraums 2 ist in der Roboterkammer 8 ein Betätigungsroboter angeordnet, welcher eine Führungseinheit 10 aufweist. Diese Führungseinheit 10 ist magnetisch mit einer Magnethalterung 12 der Hub-Greifeinheit 11 gekoppelt, so dass die Hub-Greifeinheit 11 von der Trennplatte 6 in den Inkubationsraum 2 herabhängt, ohne in diese hineinzufallen. Bewegt sich die Führungseinheit 10 in zwei Dimensionen auf der Trennplatte 6, so folgt die Magnethalterung 12 aufgrund der Magnetkraft der Bewegung der Führungseinheit 10, so dass die Hub-Greifeinheit im Inneren des Inkubationsraums 2 letztlich von außerhalb des Inkubationsraums 2 gesteuert werden kann. Dies hat den Vorteil, dass der Betätigungsroboter 8 nicht dem aggressiven Klima innerhalb des Inkubationsraums 2 ausgesetzt ist und daher in herkömmlicher Weise konstruiert sein kann. Der Betätigungsroboter ist zusammen mit der Führungseinheit 10 somit letztlich von dem Klima des Inkubationsraums 2 entkoppelt. Lediglich der in dem Inkubationsraum 2 aufgenommene Teil, also die Hub-Greifeinheit 11 selbst, ist aus Edelstahl hergestellt, um nicht zu korrodieren. Die Betätigung eines Greifers der Hub-Greifeinheit erfolgt in Form einer Höhenverfahrung und einem Öffnen und Schließen zweier einander gegenüberliegenden Greiferplatten 15, wie in Figur 3 gezeigt. Im Einzelnen wird der Greifer durch ein Auseinanderfahren der Greiferplatten 15 geöffnet, dann über einen Hubzylinder bis auf die Höhe einer Mikrotiterplatte 23 herabgelassen, durch ein aufeinander zu fahren der Greiferplatten 15 wieder geschlossen und zusammen mit der so gegriffenen Mikrotiterplatte 23 angehoben. Die Greiferplatten 15 sind an einem unteren Ende umgebogen und bilden dadurch Greifhaken aus, die unter die Mikrotiterplatten 23 geschoben werden, um diese abzustützen. Die Auslegung der Magnetkraft, welche die Hub-Greifeinheit 11 an der Führungseinheit 10 hält, muss das Gewicht einer schwerstmöglichen Mikrotiterplatte 23 berücksichtigen.

Figur 4 zeigt hierbei ein Detail der Magnethalterung 12, welche durch die Trennplatte 6 hindurch magnetisch mit der Führungseinheit 10 gekoppelt ist. Um zu vermeiden, dass Führungseinheit 10 oder Magnethalterung 12 aufgrund des Stick-Slip-Effekts während der Bewegung hängen bleiben und sich gegebenenfalls verlieren und die Hub-Greifeinheit aufgrund des sich dabei entfernenden Magnetfelds von der Trennplatte 6 herabfällt, wird versucht, den Stick-Slip-Effekt zu vermeiden. Hierzu wird ein Luftkissen 13 geschaffen, welches die Führungseinheit 10 und die Magnethalterung 12 in einen Abstand zu der Trennplatte 6 bringt, der aber dennoch gering genug ist, so dass die magnetische Kopplung erhalten bleibt. Die Führungseinheit 10 wird durch den Roboter in sehr geringen Abstand über der Trennplatte 6 mechanisch gehalten und bewegt, ohne dass die Trennplatte berührt wird.

Dieses Luftkissen 13 wird durch Ansaugen der Luft aus dem Inkubationsraum 2 erzeugt. Diese Luft wird in Richtung der Trennplatte 6 geblasen. Hierbei ist der Magnethalterung 12 ein HEPA-Filter 14 zugeordnet, durch welchen die angesaugte Inkubationsluft, also in dem Inkubationsraum 2 vorgehaltene Luft, unter den von der Steuereinheit geschaffenen Bedingungen, gefiltert wird. Der Betrieb der Magnethalterung 12 sorgt damit zusätzlich für eine Luftzirkulation und eine Luftreinigung innerhalb des Inkubationsraums 2. Der HEPA-Filter 14 kann bedarfsweise auch außerhalb des Inkubationsraums 2 platziert und über längere Luftwege eingebunden werden, so dass er leichter von außerhalb des Inkubationsraums 2 gewechselt werden kann.

Die Hub-Greifeinheit 11 ist hierbei pneumatisch betätigt und über Ventile angesteuert und weist hierfür eine Schlauchleitung auf, über die Druckluft von außerhalb des Inkubationsraums 2 von der Steuereinheit bereitgestellt wird. Für die Unterbringung weiterer mechanischer Teile ist in dem Inkubationsraum 2 eine Freiheit 26 vorgesehen, in welcher die Hub-Greifeinheit 11 nichts greifen muss. Soweit sich oberhalb dieses Bereichs in der Roboterkammer 8 diese mechanischen Teile befinden, stören diese nicht die Beweglichkeit der Hub-Greifeinheit 11. Auf diese Weise wird die Mikrotiterplatte 23 von der Lagerstelle 5 nur leicht angehoben, bis sie über den Boden 4 des Inkubationsraums hinweggehoben werden kann. Anschließend wird die Mikrotiterplatte 23 in einer Lagerstelle 21 in einem Ausschieber 20 platziert, welcher zunächst in einer Schleusenkammer 16, die ebenfalls am Boden 4 des Inkubationsraums 2 angeordnet ist, abgelegt wird. Dadurch, dass die Lagerstelle 21 in dem Ausschieber 20 in der gleichen Höhe angeordnet ist wie andere Lagerstellen 5 in dem Inkubator 1, muss die Hub-Greifeinheit 11 in der Höhe nur minimal verfahren werden.

Die Ausschleusung einer Mikrotiterplatte erfolgt über die Schleusenkammer 16, welche durch drei Türen 17, 18 und 19 verschlossen ist. Die Innentür 17 ist eine oberseitige Klappe der Schleusenkammer 16, so dass bei einer Öffnung der Klappe möglichst wenig Austausch zwischen dem Inkubationsraum 2 und der Schleusenkammer 16 entsteht. Nach dem Beladen des Ausschiebers 20 wird die Hub-Greifeinheit 11 aus einem Schwenkbereich der Innentür 17 wegbewegt und die Innentür 17 verschlossen. Die Schleusenkammer 16 ist damit zur Umgebung und zum Inkubationsraum 2 hin abgedichtet und es kann eine Flutung mit Außenluft erfolgen, bedarfsweise wird die Luft gefiltert, um einen Austritt kritischer Substanzen zu vermeiden. Sodann werden eine Außentür 18 und eine Sicherheitstür 19 geöffnet und der Ausschieber 20 betätigt, um die auszuschleusende Mikrotiterplatte 23 aus der Schleuse 28 zur Entnahme an einer Entnahmestelle zu positionieren. Je nach Grad der weiteren Automatisierung kann die Mikrotiterplatte 23 nunmehr automatisiert oder manuell aus der Lagerstelle 21 des Ausschiebers 20 entnommen und der weiteren Analyse zugeführt werden.

In umgekehrter Richtung kann eine Mikrotiterplatte 23 schließlich wieder in den Inkubator 1 eingeschleust werden. Hierbei ist zu beachten, dass die Sicherheitstür 19 im Fall einer automatischen Einschleusung entfallen kann. Sie dient dazu, Verletzungen im Fall einer manuellen Betätigung zu vermeiden. Im Fall einer manuellen Betätigung wird die Sicherheitstür 19 nach dem Einsetzen der Mikrotiterplatte 23 in den Ausschieber 20 und dessen Einrücken in die Schleusenkammer 16 zuerst geschlossen. Erst wenn die Sicherheitstür 19 geschlossen ist, was über einen Sensor erfasst wird, kann die Außentür 18 geschlossen werden, was im Gegensatz zur Sicherheitstür 19 pneumatisch erfolgt. Die Sicherheitstür 19 kann vielmehr manuell auf- und zugeklappt werden. Ist sie verschlossen, kann allerdings kein Finger oder anderes Körperteil mehr in den Eingriffsbereich der Außentür 18 einragen und bei deren Verschließen verletzt werden. Nach dem Schließen der Außentür 18 kann das Klima in der Schleusenkammer 16 an das Klima in dem Inkubationsraum 2 angepasst und die Innentür 17 geöffnet werden. Die Mikrotiterplatte 23 wird von der Hub-Greifeinheit 11 erfasst und zurück an die Lagerstelle 5 verbracht.

Ergänzend ist dem Inkubationsraum 2 ein Analysator 25 zugeordnet, welcher sich in der Steuerkammer 9 unterhalb des Bodens 4 befindet. In dem Boden ist ein Fenster aus Borosilikatglas vorgesehen, durch welches der Analysator 25 die Proben einer auf dem Fenster abgestellten Mikrotiterplatte 23 analysieren kann. In Figur 1 ist eine Mikrotiterplatte 23 auf dem Analysator 25 platziert. Für diesen Vorgang muss die Mikrotiterplatte 23 den Inkubationsraum 2 nicht verlassen, so dass diese Analyse ganz ohne Beeinträchtigung des Klimas in dem Inkubationsraum erfolgen kann. Soweit und solange diese Analyse ausreichend ist, muss der Inkubationsraum 2 mithin gar nicht geöffnet werden und kann ganz und gar automatisiert betrieben werden.

Vorstehend beschrieben ist somit ein Inkubator, der sowohl für eine effektive Sterilisierung geeignet, als auch vollständig zur Entnahme von Mikrotiterplatten automatisierbar ist.

### BEZUGSZEICHENLISTE

- 1: Inkubator
- 2: Inkubationsraum
- 3: Außenwandung
- 4: Boden
- 5: Lagerstelle
- 6: Trennplatte
- 7: Beleuchtungseinrichtung
- 8: Roboterkammer
- 9: Steuerkammer
- 10: Führungseinheit
- 11: Hub-Greifeinheit
- 12: Magnethalterung
- 13: Luftkissen
- 14: HEPA-Filter
- 15: Greiferplatte
- 16: Schleusenkammer
- 17: Innentür
- 18: Außentür
- 19: Sicherheitstür
- 20: Ausschieber
- 21: Lagerstelle
- 22: Mikrotiterplattenschüttler
- 23: Mikrotiterplatte
- 24: Dockingstation
- 25: Analysator
- 26: Freiheit
- 27: Gehäusetür
- 28: Schleuse

## Patentansprüche

1. Inkubator, umfassend einen gegenüber einer Umgebung druck- und gasdicht verschließbaren, sowie hinsichtlich der Zusammensetzung der Gase und Feuchtigkeit seiner Atmosphäre anpassbaren Inkubationsraum (2) zur Aufnahme wenigstens einer Mikrotiterplatte (23), in dem eine Hub-Greifeinheit (11) zum Transport der wenigstens einen Mikrotiterplatte (23) innerhalb des Inkubationsraums (2) beweglich gelagert ist, wobei der Inkubationsraum (2) über eine Schleusenkammer (16) zugänglich ist, welche gegenüber der Umgebung mithilfe einer Außentür (18) und gegenüber dem Inkubationsraum (2) mithilfe einer Innentür (17) verschließbar ist, wobei die Hub-Greifeinheit (11) zwischen einer ersten Position oberhalb der Schleusenkammer (16) und wenigstens einer zweiten Position oberhalb einer Lagerstelle (5) für die wenigstens eine Mikrotiterplatte (23) verfahrbar ist,
**dadurch gekennzeichnet, dass** eine Raumdecke des Inkubationsraums (2) als nichtmagnetische Trennplatte (6) ausgestaltet ist, wobei ein Betätigungsroboter zur Steuerung einer Bewegung der Hub-Greifeinheit (11) mit einer Führungseinheit (10) oberhalb der Trennplatte (6) angeordnet ist und die Hub-Greifeinheit (11) mithilfe einer durch die Trennplatte (6) hindurch wirkenden magnetischen oder elektromagnetischen Magnethalterung (12) im Inneren des Inkubationsraums (2) auf der Innenfläche der Trennplatte (6) verschieblich gehalten ist und einen höhenverstellbaren Greifer umfasst und die Führungseinheit (10) und/oder die Magnethalterung (12) Luftauslassdüsen auf einer der Trennplatte (6) zugewandten Fläche zur Ausbildung eines Luftkissens (13) zwischen der Fläche und der Trennplatte (6) aufweisen.

2. Inkubator gemäß Anspruch 1, **dadurch gekennzeichnet, dass** die Innentür (17) auf oder in oder über einem Boden (4) des Inkubationsraums (2) angeordnet und abgedichtet ist und entweder horizontal verschiebbar oder um eine horizontale Achse herum in den Inkubationsraum (2) hinein verschwenkbar ist.

3. Inkubator gemäß einem der Ansprüche 1 oder 2, **dadurch gekennzeichnet, dass** die Außentür (18) an einer Außenwandung (3) des Inkubationsraums (2) angeordnet und abgedichtet ist und entweder vertikal verschiebbar oder um eine horizontale Achse herum verschwenkbar ist.

4. Inkubator gemäß einem der vorhergehenden Ansprüche, **dadurch gekennzeichnet, dass** die Innentür (17) und die Außentür (18) selbsttätig öffnend und schließend sind, vorzugsweise durch einen elektrischen oder pneumatischen Antrieb betätigt sind.

5. Inkubator gemäß einem der vorhergehenden Ansprüche, **dadurch gekennzeichnet, dass** der Außentür (18) eine motorisch entkoppelte Sicherheitstür (19) zugeordnet ist, deren Verschlussposition sensorisch erfasst ist, wobei eine Schließung der Außentür (18) nur dann ermöglicht ist, wenn die Sicherheitstür (19) verschlossen ist.

6. Inkubator gemäß einem der Ansprüche 1 bis 4, **dadurch gekennzeichnet, dass** eine Fördereinrichtung vorgesehen ist, welche der Schleusenkammer (16) die Mikrotiterplatten (23) selbsttätig zuführt.

7. Inkubator gemäß einem der vorhergehenden Ansprüche, **dadurch gekennzeichnet, dass** der Schleusenkammer (16) ein Ausschieber (20) zugeordnet ist, welcher eine Lagerstelle (21) für wenigstens eine Mikrotiterplatte (23) ausbildet und aus der Schleusenkammer (16) heraus durch die Außentür (18), vorzugsweise mithilfe eines elektrischen oder pneumatischen Antriebs, in eine Zugriffsposition verfahrbar ist.

8. Inkubator gemäß einem der vorhergehenden Ansprüche, **dadurch gekennzeichnet, dass** die Luft zur Erzeugung des Luftkissens aufseiten der Magnethalterung (12) aus dem Inkubationsraum (2) angesaugt und vorzugsweise durch ein HEPA-Filter (14) geleitet und/oder durch eine Peltier-Kühlung gekühlt wird.

9. Inkubator gemäß einem der vorhergehenden Ansprüche , **dadurch gekennzeichnet, dass** die Hub-Greifeinheit (11) einen elektrisch betriebenen Greifer aufweist, welcher über die Magnethalterung (12) induktiv mit einer Betriebsspannung versorgt ist.

10. Inkubator gemäß einem der Ansprüche 1 bis 9, **dadurch gekennzeichnet, dass** die Hub-Greifeinheit (11) einen pneumatisch betriebenen Greifer aufweist, welcher mithilfe pneumatischer Schlauchleitungen, welche an wenigstens einer Außenwandung (3), dem Boden (4) oder der Trennplatte (6) in den Inkubationsraum (2) münden und von außerhalb des Inkubationsraums (2) beschickt sind, mit Druckluft versorgt ist.

11. Inkubator gemäß einem der vorhergehenden Ansprüche, **dadurch gekennzeichnet, dass** die Trennplatte (6) als Glasplatte, vorzugsweise aus Borosilikatglas, ausgestaltet ist.

12. Inkubator gemäß Anspruch 11, **dadurch gekennzeichnet, dass** der Inkubationsraum (2) durch die Glasplatte hindurch mithilfe einer außerhalb des Inkubationsraums (2) angeordneten Beleuchtungseinrichtung (7) beleuchtet ist.

13. Inkubator gemäß einem der vorhergehenden Ansprüche, **dadurch gekennzeichnet, dass** die Hub-Greifeinheit (11) zumindest im Wesentlichen aus einem nichtkorrosiven Material, vorzugsweise aus Edelstahl, hergestellt ist.

14. Inkubator gemäß einem der vorhergehenden Ansprüche, **dadurch gekennzeichnet, dass** der Schleusenkammer (16) Mittel zur selbsttätigen Identifikation einzelner Mikrotiterplatten (23) zugeordnet sind, insbesondere ein Barcodeleser oder ein Nahfeldkommunikationsleser.

15. Inkubator gemäß einem der vorhergehenden Ansprüche, **dadurch gekennzeichnet, dass** dem Boden (4) des Inkubationsraums (2) wenigstens ein Mikrotiterplattenschüttler (22), vorzugsweise eine Mehrzahl von rasterförmig angeordneten Mikrotiterplattenschüttlern (22), zugeordnet ist, wobei der wenigstens eine Mikrotiterplattenschüttler (22) vorzugsweise im Rundlauf und/oder im Längslauf betreibbar ist.

16. Inkubator gemäß Anspruch 15, **dadurch gekennzeichnet, dass** der wenigstens eine Mikrotiterplattenschüttler (22) auf einer Basisplatte aufgesteckt ist, die zur Reinigung und/oder Desinfektion mit sämtlichen Mikrotiterplattenschüttlern (22) als Ganzes aus dem Inkubationsraum (2) entnehmbar ist.

17. Inkubator gemäß einem der Ansprüche 15 oder 16, **dadurch gekennzeichnet, dass** der wenigstens eine Mikrotiterplattenschüttler (22) eine Lagerstelle (21) zur zentrierten und vorzugsweise kraft- oder reibschlüssigen Aufnahme der wenigstens einen Mikrotiterplatte (23) aufweist, wobei vorzugsweise die Lagerhöhe einer Mikrotiterplatte (23) in der Lagerstelle (21) des wenigstens einen Mikrotiterplattenschüttlers (22) der Lagerhöhe der Mikrotiterplatte (23) in der Schleusenkammer (16) zumindest näherungsweise entspricht.

18. Inkubator gemäß einem der vorhergehenden Ansprüche, **dadurch gekennzeichnet, dass** dem Boden (4) des Inkubationsraums (2) wenigstens ein Analysator (25) zur Auswertung von Proben in einer auf einem dem Analysator im Boden (4) freigehaltenen Fenster abgestellten Mikrotiterplatte (23) zugeordnet ist.

## Claims

1. Incubator comprising an incubation chamber (2) which can be sealed against the environment in a pressure-tight and gas-tight manner and whose composition of gases and humidity can be adjusted, for accommodating at least one microtiter plate (23), in which a lifting gripper unit (11) for transporting the at least one microtiter plate (23) is movably mounted within the incubation chamber (2),
wherein the incubation chamber (2) is accessible via an airlock chamber (16) which can be sealed off from the environment by means of an outer door (18) and from the incubation chamber (2) by means of an inner door (17), wherein the lifting gripper unit (11) is movable between a first position above the airlock chamber (16) and at least one second position above a storage location (5) for the at least one microtiter plate (23),
**characterised in that** a ceiling of the incubation chamber (2) is designed as a nonmagnetic separating plate (6), wherein an actuating robot for controlling the movement of the lifting gripping unit (11) is arranged with a guide unit (10) above the separating plate (6) and the lifting gripping unit (11) is held in a displaceable manner inside the incubation chamber (2) on the inner surface of the separating plate (6) by means of a magnetic or electromagnetic magnetic holder (12) acting through the separating plate (6) and comprises a height-adjustable gripper, and the guide unit (10) and/or the magnetic holder (12) have air outlet nozzles on a surface facing the separating plate (6) to form an air cushion (13) between the surface and the separating plate (6).

2. Incubator according to claim 1, **characterised in that** the inner door (17) is arranged and sealed on or in or above a floor (4) of the incubation chamber ( ) (2) and can either be moved horizontally or pivoted around a horizontal axis into the incubation chamber (2).

3. Incubator according to one of claims 1 or 2, **characterised in that** the outer door (18) is arranged and sealed on an outer wall (3) of the incubation chamber (2) and can either be moved vertically or pivoted around a horizontal axis.

4. Incubator according to one of the preceding claims, **characterised in that** the inner door (17) and the outer door (18) open and close automatically, preferably being actuated by an electric or pneumatic drive.

5. Incubator according to one of the preceding claims, **characterised in that** the outer door (18) is assigned a motorically decoupled safety door (19), the closed position of which is detected by a sensor, whereby closure of the outer door (18) is only possible when the safety door (19) is closed.

6. Incubator according to one of claims 1 to 4, **characterised in that** a conveyor device is provided which automatically feeds the microtiter plates (23) to the airlock chamber (16).

7. Incubator according to one of the preceding claims, **characterised in that** the airlock chamber (16) is assigned an ejector (20) which forms a storage location (21) for at least one microtiter plate (23) and can be moved out of the airlock chamber (16) through the outer door (18), preferably by means of an electric or pneumatic drive.

8. Incubator according to one of the preceding claims, **characterised in that** the air for generating the air cushion on the side of the magnetic holder (12) is sucked in from the incubation chamber (2) and preferably passed through a HEPA filter (14) and/or cooled by Peltier cooling.

9. Incubator according to one of the preceding claims, **characterised in that** the lifting gripper unit (11) has an electrically operated gripper which is supplied with operating voltage inductively via the magnetic holder (12).

10. Incubator according to one of claims 1 to 9, **characterised in that** the lifting gripper unit (11) has a pneumatically operated gripper which, with the aid of pneumatic hose lines connected to at least one outer wall (3), the base (4) or the partition plate (6) into the incubation chamber (2) and are fed from outside the incubation chamber (2).

11. Incubator according to one of the preceding claims, **characterised in that** the partition plate (6) is designed as a glass plate, preferably made of borosilicate glass.

12. Incubator according to claim 11, **characterised in that** the incubation chamber (2) is illuminated through the glass plate by means of a lighting device (7) arranged outside the incubation chamber (2).

13. Incubator according to one of the preceding claims, **characterised in that** the lifting gripping unit (11) is made at least essentially of a non-corrosive material, preferably stainless steel.

14. Incubator according to one of the preceding claims, **characterised in that** the airlock chamber (16) is assigned means for automatic identification of individual microtiter plates (23), in particular a barcode reader or a near-field communication reader.

15. Incubator according to one of the preceding claims, **characterised in that** at least one microtiter plate shaker (22), preferably a plurality of microtiter plate shakers (22) arranged in a grid pattern, are associated with the bottom (4) of the incubation chamber (2), wherein the at least one microtiter plate shaker (22) is preferably operable in circular motion and/or longitudinal motion.

16. Incubator according to claim 15, **characterised in that** the at least one microtiter plate shaker (22) is mounted on a base plate which can be removed from the incubation chamber (2) as a whole together with all microtiter plate shakers (22) for cleaning and/or disinfection.

17. Incubator according to one of claims 15 or 16, **characterised in that** the at least one microtiter plate shaker (22) has a bearing point (21) for centred and preferably force- or friction-locking of the at least one microtiter plate (23), wherein preferably the bearing height of a microtiter plate (23) in the bearing point (21) of the at least one microtiter plate (23) is 1.5 mm.or friction-locked mounting of the at least one microtiter plate (23), wherein preferably the bearing height of a microtiter plate (23) in the bearing location (21) of the at least one microtiter plate shaker (22) corresponds at least approximately to the bearing height of the microtiter plate (23) in the airlock chamber (16).

18. Incubator according to one of the preceding claims, **characterised in that** the bottom (4) of the incubation chamber (2) is at lea ly assigned an analyser (25) for evaluating samples in a microtiter plate (23) placed on a window kept free on the analyser in the bottom (4).

## Revendications

1. Incubateur comprenant une chambre d'incubation (2) pouvant être fermée de manière étanche à la pression et aux gaz par rapport à un environnement et pouvant être adaptée en termes de composition des gaz et d'humidité de son atmosphère, destinée à recevoir au moins une plaque de microtitrage (23), dans laquelle une unité de préhension à levage (11) est montée de manière mobile pour transporter au moins une plaque de microtitrage (23) à l'intérieur de la chambre d'incubation (2),
la chambre d'incubation (2) est accessible via une chambre de sas (16) qui peut être fermée par rapport à l'environnement à l'aide d'une porte extérieure (18) et par rapport à la chambre d'incubation (2) à l'aide d'une porte intérieure (17), l'unité de préhension à levage (11) pouvant être déplacée entre une première position au-dessus de la chambre de sas (16) et au moins une deuxième position au-dessus d'un emplacement de stockage (5) pour au moins une plaque de microtitrage (23),
**caractérisé en ce qu'**un plafond de la chambre d'incubation (2) est conçu comme une plaque de séparation non magnétique (6), un robot d'actionnement destiné à commander un mouvement de l'unité de préhension à levage (11) étant disposé avec une unité de guidage (10) au-dessus de la plaque de séparation (6) et l'unité de préhension à levage (11) est maintenue de manière mobile à l'intérieur de la chambre d'incubation (2) sur la surface intérieure de la plaque de séparation (6) à l'aide d'un support magnétique (12) magnétique ou électromagnétique agissant à travers la plaque de séparation (6) et comprend un préhenseur réglable en hauteur, et l'unité de guidage (10) et/ou le support magnétique (12) comportent des buses de sortie d'air sur une surface tournée vers la plaque de séparation (6) afin de former un coussin d'air (13) entre la surface et la plaque de séparation (6).

2. Incubateur selon la revendication 1, **caractérisé en ce que** la porte intérieure (17) est disposée et étanchée sur ou dans ou au-dessus d'un fond (4) de la chambre d'incubation (2) et peut être déplacée horizontalement ou pivotée de manière ement autour d'un axe horizontal dans la chambre d'incubation (2).

3. Incubateur selon l'une des revendications 1 ou 2, **caractérisé en ce que** la porte extérieure (18) est disposée et étanchée sur une paroi extérieure (3) de la chambre d'incubation (2) et peut être déplacée verticalement ou pivotée autour d'un axe horizontal.

4. Incubateur selon l'une des revendications précédentes, **caractérisé en ce que** la porte intérieure (17) et la porte extérieure (18) s'ouvrent et se ferment automatiquement, de préférence actionnées par un entraînement électrique ou pneumatique.

5. Incubateur selon l'une des revendications précédentes, **caractérisé en ce qu'**une porte de sécurité (19) découplée du moteur est associée à la porte extérieure (18), dont la position de fermeture est détectée par un capteur, la fermeture de la porte extérieure (18) n'étant possible que lorsque la porte de sécurité (19) est fermée.

6. Incubateur selon l'une des revendications 1 à 4, **caractérisé en ce qu'**il est prévu un dispositif de transport qui achemine automatiquement les plaques de microtitrage (23) vers la chambre de sas (16).

7. Incubateur selon l'une des revendications précédentes, **caractérisé en ce qu'**un dispositif d'éjection (20) est associé à la chambre de sas (16), lequel forme un emplacement de stockage (21) pour au moins une plaque de microtitrage (23) et peut être déplacé hors de la chambre de sas (16) à travers la porte extérieure (18), de préférence à l'aide d'un entraînement électrique ou pneumatique, dans une position d'accès.

8. Incubateur selon l'une des revendications précédentes, **caractérisé en ce que** l'air destiné à la génération du coussin d'air est aspiré du côté du support magnétique (12) à partir de la chambre d'incubation (2) et est de préférence acheminé à travers un filtre HEPA (14) et/ou refroidi par un refroidissement Peltier.

9. Incubateur selon l'une des revendications précédentes, **caractérisé en ce que** l'unité de préhension à levage (11) comporte une pince à commande électrique qui est alimentée en tension de service par induction via le support magnétique (12).

10. Incubateur selon l'une des revendications 1 à 9, **caractérisé en ce que** l'unité de préhension à levage (11) comporte une pince à commande pneumatique qui est alimentée en air comprimé à l'aide de tuyaux pneumatiques qui débouchent dans la chambre d'incubation (2) au niveau d'au moins une paroi extérieure (3), au fond (4) ou à la plaque de séparation (6) dans l'espace d'incubation (2) et alimentés depuis l'extérieur de l'espace d'incubation (2).

11. Incubateur selon l'une des revendications précédentes, **caractérisé en ce que** la plaque de séparation (6) est conçue comme une plaque de verre, de préférence en verre borosilicaté.

12. Incubateur selon la revendication 11, **caractérisé en ce que** la chambre d'incubation (2) est éclairée à travers la plaque de verre à l'aide d'un dispositif d'éclairage (7) disposé à l'extérieur de la chambre d'incubation (2).

13. Incubateur selon l'une des revendications précédentes, **caractérisé en ce que** l'unité de préhension à levage (11) est fabriquée au moins essentiellement dans un matériau non corrosif, de préférence en acier inoxydable.

14. Incubateur selon l'une des revendications précédentes, **caractérisé en ce que** la chambre de sas (16) est associée à des moyens d' dentification automatique de plaques de microtitrage individuelles (23), en particulier un lecteur de codes-barres ou un lecteur de communication en champ proche.

15. Incubateur selon l'une des revendications précédentes, **caractérisé en ce qu'**au moins un agitateur de plaques de microtitrage (22), de préférence une pluralité d'agitateurs de plaques de microtitrage (22) disposés en forme de grille, étant associé au fond (4) de la chambre d'incubation (2), le au moins un agitateur de plaques de microtitrage (22) pouvant de préférence fonctionner en rotation et/ou en translation.

16. Incubateur selon la revendication 15, **caractérisé en ce que** le au moins un agitateur de plaques de microtitrage (22) est monté sur une plaque de base qui peut être retirée de la chambre d'incubation (2) avec tous les agitateurs de plaques de microtitrage (22) dans son ensemble à des fins de nettoyage et/ou de désinfection.

17. Incubateur selon l'une des revendications 15 ou 16, **caractérisé en ce que** le au moins un agitateur de plaques de microtitrage (22) présente un palier (21) pour le logement centré et de préférence à forceou par friction de la au moins une plaque de microtitrage (23), la hauteur de support d'une plaque de microtitrage (23) dans le point d'appui (21) du au moins un agitateur de plaques de microtitrage (22) correspondant de préférence au moins approximativement à la hauteur de support de la plaque de microtitrage (23) dans la chambre de sas (16).

18. Incubateur selon l'une des revendications précédentes, **caractérisé en ce qu'**au moins un analyseur (25) est associé au fond (4) de la chambre d'incubation (2) pour évaluer des échantillons dans une plaque de microtitrage (23) placée sur une fenêtre laissée libre dans le fond (4) de l'analyseur.
